# EUROPÄISCHE PATENTSCHRIFT

(11) **EP 3 737 441 B1**
(45) Veröffentlichungstag und Bekanntmachung des Hinweises auf die Patenterteilung: **19.06.2024**
(21) Anmeldenummer: 19700345.2
(22) Anmeldetag: 08.01.2019
(51) Int. Cl.: A61M 1/14, E03C 1/12

(54) **VORRICHTUNG ZUM ABLEITEN VON ABWASSER**
DEVICE FOR DISCHARGING WASTE WATER
APPAREIL POUR L'ÉVACUATION DES EAUX USÉES

(30) Priorität: 11.01.2018 DE 102018000146
(43) Veröffentlichungstag der Anmeldung: 18.11.2020
(73) Patentinhaber: Fresenius Medical Care Deutschland GmbH, 61352 Bad Homburg v.d.H. (DE)
(72) Erfinder: ENGELN, Florian, 35321 Laubach (DE)
(74) Vertreter: Oppermann, Frank
(86) Internationale Anmeldenummer: PCT/EP2019/050281
(87) Internationale Veröffentlichungsnummer: WO 2019/137885

(56) Entgegenhaltungen:
- EP-A1- 2 286 850
- US-A- 4 176 684

## Beschreibung

Die Erfindung betrifft eine Vorrichtung zum Ableiten von Abwasser, insbesondere von gebrauchter oder verbrauchter Dialysierflüssigkeit, die eine Mehrzahl von Leitungsanschlüssen zum Anschluss von Abwasserleitungen, insbesondere Abwasserleitungen von Dialysegeräten, aufweist.

Der Einsatz von medizintechnischen Geräten in der Klinik setzt vielfach die Ableitung von gebrauchten oder verbrauchten Flüssigkeiten in einen Ablauf voraus. Diese Flüssigkeiten werden nachfolgend als Abwasser bezeichnet.

Die bekannten Dialysegeräte beispielsweise verfügen über Abwasserschläuche, um gebrauchte bzw. verbrauchte Dialysierflüssigkeit abzuleiten. Beim Ableiten der Dialysierflüssigkeit ist zu beachten, dass der Abwasserschlauch mit dem Abwasserleitungssystem nicht in direkter Flüssigkeitsverbindung stehen darf, da ansonsten die Gefahr einer Kontamination besteht. Dies kann dadurch erreicht werden, dass das freie Ende des Abwasserschlauchs nicht direkt an ein Abwasserleitungssystem angeschlossen wird, sondern eine definierte Freifallstrecke eingehalten wird. Das freie Ende des Abwasserschlauchs kann beispielsweise in einer definierten Höhe über dem Boden eines Abflussbeckens (Waschbeckens) angeordnet werden.

In der Praxis stellt sich das Problem einer ausreichenden Führung und Fixierung der Abwasserleitung unter gleichzeitiger Einhaltung einer definierten Freifallstrecke. Dieses Problem stellt sich sowohl in der chronischen als auch der Akutdialyse. Insbesondere erweist sich die Führung und Fixierung mehrerer Abwasserleitungen beim Einsatz von mehreren Dialysegeräten auf einer Station als problematisch.

Im Bereich der Haushalts- oder Sanitärtechnik ist das Prinzip der Bildung einer Freifallstrecke aus der US 7 290 557 B1 bekannt. Allerdings stellt sich in der Haushalts- oder Sanitärtechnik nicht das Problem der Kontamination medizinischer Flüssigkeiten oder medizintechnischer Einrichtungen. Die US 7 290 557 B1 beschreibt ein Anschlussstück für den Anschluss eines Brauchwasserschlauchs einer Spülmaschine an das Fallrohr eines Brauchwasserleitungssystems. Das Anschlussstück, das mittels einer Schraub- oder Steckverbindung mit einem Fallrohr fest verbunden wird, weist ein Rohrstück auf, durch das das Brauchwasser in das Fallrohr strömt. Zu besseren Be- bzw. Entlüftung weist dieses Rohrstück eine seitliche Öffnung auf. Anschlussstück und Fallrohr haben im Wesentlichen den gleichen Durchmesser. Das Anschlussstück besteht vorzugsweise aus Kunststoff.

Nachteilig ist, dass das Anschlussstück nur den Anschluss eines einzigen Brauchwasserschlauchs an ein Fallrohr erlaubt. Für den Anschluss von mehreren Brauchwasserschläuchen sind mehrere Anschlusstücke erforderlich, wobei an dem Fallrohr entsprechende Verzweigungen erforderlich sind, an denen die Anschlusstücke angeschlossen werden können. Dies erfordert einen erhöhten Platzbedarf.

Darüber hinaus ist im Bereich der Haushaltstechnik die Fixierung einer Brauchwasserleitung, beispielsweise des Brauchwasserschlauchs einer Waschmaschine, an dem Beckenrand eines Waschbeckens bekannt.

Die EP 2 286 850 A1 beschreibt eine Vorrichtung zum Ableiten von Abwasser für Dialysegeräte, welche eine Mehrzahl von Leitungsanschlüssen zum Anschluss von Abwasserleitungen des Dialysegeräts aufweist. Die Vorrichtung zum Ableiten von Abwasser ist als ein Behälter ausgebildet, der zur Montage an einem Wandbefestigungsteil ausgebildet ist. Der Behälter weist einen trichterförmigen Teil auf, der von einem Deckelteil verschlossen wird. An dem Deckelteil sind die Leitungsanschlüsse für die Abwasserleitungen des Dialysegeräts vorgesehen. Die Leitungsanschlüsse bilden eine Freifallstrecke für das Abwasser vom Deckelteil bis zu der schrägen Wand des trichterförmigen Teils des Behälters.

Die US 4 176 684 A befasst sich allgemein mit dem Ableiten von Abwasser in der Haushaltstechnik, insbesondere mit dem Ableiten von Abwasser einer Waschmaschine oder eines Geschirrspülers in ein Spülbecken, wobei die Aufgabe der US 4 176 684 A allein in dem Spritzschutz liegt. Die Vorrichtung ist als ein trichterförmiger Teil ausgebildet, welcher auf den Boden des Spülbeckens aufgestellt werden soll. Der trichterförmige Teil verfügt über einen in der Höhe einstellbaren Anschlussteil, der in den Zulauf des trichterförmigen Teils geschraubt ist. Der Anschlussteil erlaubt eine Verbindung mit einer konventionellen Armatur einer Waschmaschine oder eines Geschirrspülers, welche Leitungsanschlüsse zum Zuführen von Frischwasser und Ableiten von Abwasser umfasst.

Der Erfindung liegt die Aufgabe zugrunde, die Ableitung von Abwasser, insbesondere von gebrauchter oder verbrauchter Dialysierflüssigkeit, zu vereinfachen, ohne dass die Gefahr einer Kontamination besteht.

Die Lösung dieser Aufgabe erfolgt erfindungsgemäß mit den Merkmalen des Patentanspruchs 1. Die abhängigen Ansprüche betreffen bevorzugte Ausführungsformen der Erfindung.

Die erfindungsgemäße Vorrichtung zum Ableiten von Abwasser ist insbesondere für medizinische Geräte, insbesondere Dialysegeräte, bestimmt, um medizinische Flüssigkeiten ableiten zu können. Die bevorzugte Verwendung liegt also nicht in der Versorgungstechnik (Haus- oder Gebäudetechnik) mit technischen Installationen für die Abwasserentsorgung. Die erfindungsgemäße Vorrichtung zum Ableiten von Abwasser, insbesondere von gebrauchter oder verbrauchter Dialysierflüssigkeit, verfügt über eine Mehrzahl von Leitungsanschlüssen zum Anschluss von Abwasserleitungen, insbesondere Abwasserleitungen von Dialysegeräten. Die Vorrichtung kann beispielsweise zwei oder drei oder mehr als drei Leitungsanschlüsse aufweisen, so dass die Abwasserleitungen einer Mehrzahl von Geräten, insbesondere Dialysegeräten, angeschlossen werden können.

Das Grundprinzip der Erfindung liegt darin, dass die erfindungsgemäße Vorrichtung zum Ableiten von Abwasser als ein auf den Boden eines Beckens aufstellbares Teil ausgebildet ist. Die Vorrichtung bildet daher einen einfach zu handhabenden Standfuß, der sich auf den Boden eines Beckens aufstellen lässt. Die Vorrichtung erlaubt den Anschluss mehrerer Abwasserleitungen und erfordert dabei nur einen geringen Platzbedarf. Sie kann auch seitliche Zugkräfte der Anschlussleitungen aufnehmen.

Eine ausreichende Standfestigkeit kann allein aufgrund des Gewichts (Schwerkraft) der Vorrichtung erreicht werden, ohne dass eine Befestigung der Vorrichtung beispielsweise am Rand eines Beckens erforderlich wäre. Daher kann die Vorrichtung ohne eine weitere Fixierung auch in der Mitte des Beckens platziert werden.

Eine Kontamination wird dadurch wirkungsvoll verhindert, dass die Leitungsanschlüsse mit mindestens einer Zustromleitung in Fluidverbindung stehen, deren unteres Ende unter Bildung einer Freifallstrecke im Abstand zu der Unterseite bzw. Standfläche des auf den Boden des Beckens aufstellbaren Teils angeordnet ist. Die Länge der Freifallstrecke wird durch die Höhe des unteren Endes der Zustromleitung bzw. der Abmessungen der Vorrichtung bestimmt. Aufgrund der Freifallstrecke kann das Abwasser auch unter relativ hohem Druck, der beispielsweise zwischen 2 und 4 bar liegen kann, ohne die Gefahr einer Kontamination abgeleitet werden.

Die Vorrichtung zum Ableiten von Abwasser weist ein Mantelteil auf, das die mindestens eine Zustromleitung umschließt. Das Mantelteil bildet einen Spritzschutz in Form einer Haube, so dass das unter relativ hohem Druck aus der Zustromleitung austretende Abwasser nicht in die Umgebung gelangen kann. Allein aufgrund des Gewichts des Mantelteils kann eine ausreichende Standfestigkeit erreicht werden.

Der Mantelteil weist vorzugsweise eine Mehrzahl von seitlich abstehenden Standbeinen auf, mit denen sich die Vorrichtung seitlich am Boden des Beckens sicher abstützen kann, so dass die Vorrichtung selbst dann nicht kippen oder umfallen kann, wenn allein das Eigengewicht für eine ausreichende Standfestigkeit nicht ausreichen sollte. Die Aufstandsfläche ist somit ein Vielfaches größer als der Durchmesser der Abwasserleitungen. Zusätzliche Standbeine brauchen aber nicht vorgesehen zu sein.

Eine besonders bevorzugte Ausführungsform sieht vor, dass die Standbeine Standfüße aufweisen, so dass die Unterseite des Mantelteils im Abstand zum Boden des Beckens angeordnet ist. Diese Ausführungsform hat den Vorteil, dass eine Freifallstrecke auch für an der Wandung des Mantelteils abfließendes Spritzwasser geschaffen wird. Die Länge der Freifallstrecke, d. h. des Abstandes zwischen der unteren Kante des Mantelteils und dem Boden wird durch die Abmessungen der Standfüße. bestimmt. Die Standfüße ermöglichen einen freier Abfluss von Abwasser unter dem Mantelteil zu allen Seiten.

Bei einer weiteren Ausführungsform weist die Vorrichtung ein Deckelteil auf, an dem die Leitungsanschlüsse vorgesehen sind. Der Deckelteil und der Mantelteil können zwei fest miteinander verbundene Teile oder einstückig gefertigt sein. Der Deckelteil ist vorzugsweise ein kreisförmiger oder tellerförmiger Teil, während der Mantelteil vorzugsweise ein hohlzylindrischer Teil ist. Deckelteil und Mantelteil können aber auch andere Formen haben. Beispielsweise kann der Deckelteil halbkugelförmig und der Mantelteil zylindrisch sein.

Die mindestens eine Zustromleitung kann unterschiedlich ausgebildet und angeordnet sein, beispielsweise kann die mindestens eine Zustromleitung ein Röhrchen sein. Das Röhrchen ist vorzugsweise ein gerades Röhrchen, kann aber auch gebogen sein. Das Röhrchen braucht nicht senkrecht angeordnet zu sein, sondern kann auch schräg angeordnet sein.

Das obere Ende der Zustromleitung ist vorzugsweise als Anschlussstutzen zum Anschluss einer Schlauchleitung ausgebildet. Der Anschlussstutzen ist vorzugsweise abgewinkelt, so dass die Abflussleitung von der Seite herangeführt und seitlich angeschlossen werden kann.

Eine bevorzugte Ausführungsform sieht vor, dass ein Leitungsanschluss jeweils mit einer Zustromleitung in Flüssigkeitsverbindung steht. Es ist aber auch möglich, dass sämtliche Leitungsanschlüsse mit nur einer Zustromleitung in Flüssigkeitsverbindung stehen. Einzelne Leitungsanschlüsse können auch mit mehreren Zustromleitungen in Flüssigkeitsverbindung stehen.

Die Zustromleitungen können die gleiche Länge haben, so dass sie den gleichen Abstand zu der Unterseite des auf den Boden des Beckens aufstellbaren Teils haben oder die Zustromleitungen können unterschiedliche Längen haben, so dass sie unterschiedliche Abstände zu der Unterseite des auf den Boden des Beckens aufstellbaren Teils haben. Die Zustromleitungen können den gleichen Durchmesser oder unterschiedliche Durchmesser haben. Die Länge und die Durchmesser der Zustromleitungen können somit an unterschiedliche Flussraten oder unterschiedliche Drücke der einzelnen Geräte individuell angepasst werden.

Der Deckelteil und/oder der Mantelteil bestehen vorzugsweise aus rostfreiem Stahl (Edelstahl), der leicht zu reinigen bzw. desinfizieren ist. Wenn Deckelteil und/oder Mantelteil aus Edelstahl bestehen, hat die Vorrichtung auch ein ausreichendes Eigengewicht, so dass eine ausreichende Standfestigkeit gegeben ist. Die beiden Edelstahlteile können einfach miteinander verbunden, beispielsweise verschweißt, aber auch verklebt werden. Folglich ist es nicht erforderlich, den Spritzschutz aus einem Teil zu fertigen. Wenn die Standbeine eine ausreichende Standfestigkeit sicherstellen, können Deckelteil und/oder Mantelteil grundsätzlich aber auch aus Kunststoff bestehen.

In der Praxis liegen bei bestimmten Anwendungen relative hohe Flüsse vor, so dass das Abwasser, insbesondere die Dialysierflüssigkeit mit hoher Strömungsgeschwindigkeit, beispielsweise 6 m/s, aus der Zustromleitung in die Freifallstrecke eintritt, wodurch es zu unerwünschtem Spritzen kommen kann.

Eine weitere Ausführungsform sieht vor, dass die Zustromleitung oder mindestens eine der Zustromleitungen einen Diffusor aufweist oder als Diffusor ausgebildet ist, so dass die Flüssigkeitsströmung verlangsamt wird. Anstelle der Zustromleitung kann auch der zugehörige Leitungsanschluss einen Diffusor aufweisen oder als Diffusor ausgebildet sein. Bei einer bevorzugten Ausführungsform weist die Zustromleitung oder mindestens eine der Zustromleitungen einen zum unteren Ende aufweitenden Querschnitt auf. Der Querschnitt kann sich über die gesamte Länge oder nur über einen Teil der Länge, beispielsweise auch nur am unteren Endstück der Zustromleitung vergrößern. Aufgrund der geringeren Strömungsgeschwindigkeit des Abwassers, insbesondere der Dialysierflüssigkeit, wird unerwünschtes Spritzen zumindest vermindert oder verringert.

Nachfolgend werden Ausführungsbeispiele der erfindungsgemäßen Vorrichtung zum Ableiten von Abwasser unter Bezugnahme auf die Figuren im Einzelnen beschrieben.

Es zeigen:
- Fig. 1: ein Ausführungsbeispiel der erfindungsgemäßen Vorrichtung in perspektivischer Darstellung,
- Fig. 2: die Vorrichtung von Fig. 1 in der Draufsicht,
- Fig. 3: einen Schnitt durch die Vorrichtung von Fig. 1,
- Fig. 4: ein zweites Ausführungsbeispiel der Vorrichtung in geschnittener Darstellung,
- Fig. 5: ein drittes Ausführungsbeispiel der Vorrichtung in geschnittener Darstellung,
- Fig. 6: eine perspektivische Darstellung der Vorrichtung, wobei die Abwasserschläuche an die Vorrichtung angeschlossen sind, und
- Fig. 7: ein weiteres Ausführungsbeispiel einer Zustromleitung.

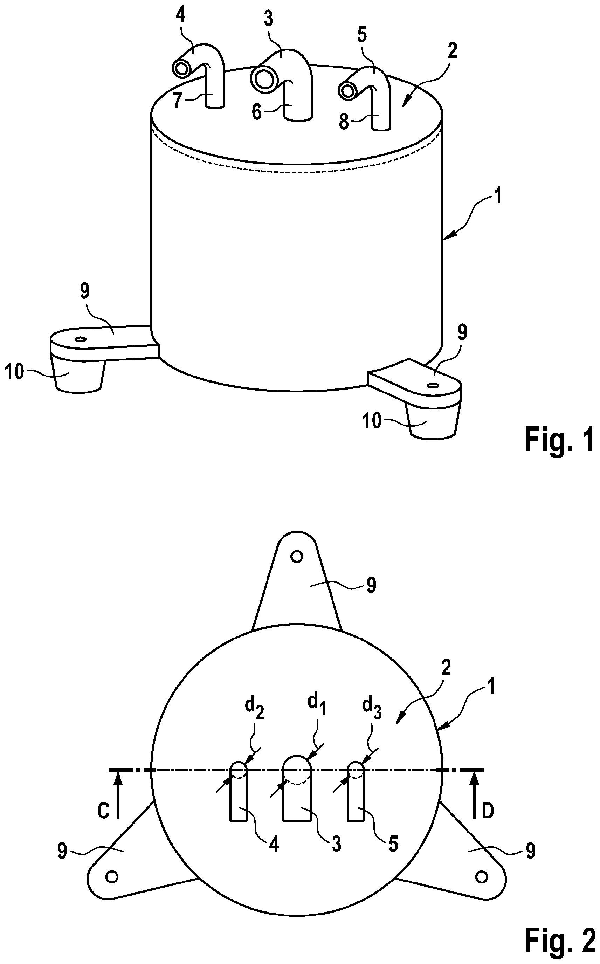

Fig. 1 zeigt eine perspektivische Darstellung der erfindungsgemäßen Vorrichtung zum Ableiten von Abwasser. Fig. 2 zeigt die Vorrichtung in der Draufsicht. Die Vorrichtung weist einen hohlzylindrischen Mantelteil 1 auf, der an der Oberseite von einem kreisförmigen Deckelteil 2 verschlossen ist. Mantelteil 1 und Deckelteil 2 bestehen aus rostfreiem Edelstahl und sind zu einem haubenförmigen Körper, der auch als Glocke bezeichnet werden kann, miteinander verbunden, insbesondere verschweißt.

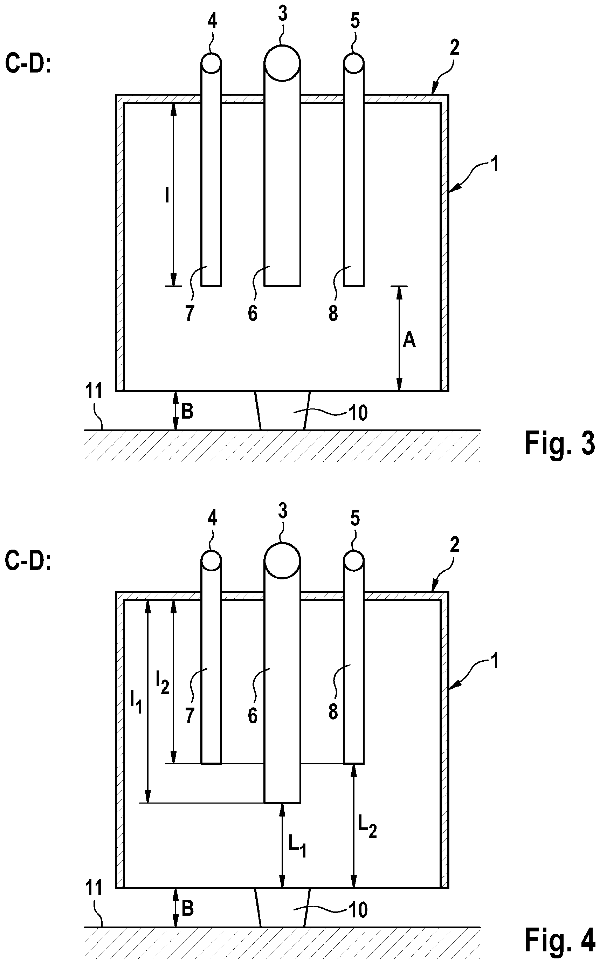

An dem Deckelteil 2 sind drei im Abstand zueinander angeordnete Leitungsanschlüsse 3, 4, 5 zum Anschluss von nicht dargestellten Schlauchleitungen vorgesehen. Die Leitungsanschlüsse 3, 4, 5 sind als Anschlussstutzen ausgebildet, auf die sich Abwasserschläuche von medizintechnischen Geräten, insbesondere Dialysegeräten, passend aufschieben lassen. Anstelle der Anschlussstutzen können aber auch Schlauchleitungskupplungen oder dergleichen vorgesehen sein, um die Schlauchleitungen einfach anschließen bzw. abnehmen zu können. Die Anschlussstutzen 3, 4, 5 sind gegenüber der Achse des zylindrischen Mantelteils 1 um 90° abgewinkelt, so dass sich die Abwasserschläuche seitlich aufschieben lassen. Der mittlere Anschlussstutzen 3 hat einen Außendurchmesser d₁, der größer ist als die Außendurchmesser d₂, d₃ der äußeren Anschlussstutzen 4, 5, so dass auf den mittleren Anschlussstutzen 3 eine Abwasserschlauch mit einem größeren Durchmesser passend aufgeschoben werden kann. Die Anschlussstutzen sind mit Zustromleitungen 6, 7, 8 einstückig, die sich in das Innere des Mantelteils 1 erstrecken. Die Zustromleitungen 6, 7, 8 mit den Anschlussstutzen 3, 4, 5 bestehen vorzugsweise aus Edelstahl (Fig. 3).

Fig. 3 zeigt einen Schnitt durch den Mantelteil 1. Die Zustromleitungen 6, 7, 8 sind gerade Röhrchen, die sich von dem Deckelteil 2 nach unten erstrecken. Die mittlere Zustromleitung 6 erstreckt sich auf der Längsachse und die beiden äußeren Zustromleitungen 7, 8 neben der Längsachse des zylindrischen Mantelteils 1. Die Zustromleitungen 6, 7, 8 haben die gleiche Länge 1. Die unteren Enden der Zustromleitungen 6, 7, 8 sind im Abstand zu der unteren Kante des Mantelteils 1 angeordnet, so dass für das aus den Zustromleitungen 6, 7, 8 austretende Abwasser eine Freifallstrecke ausgebildet wird. Dieser Abstand ist in Fig. 3 mit dem Bezugszeichen A bezeichnet.

Der Mantelteil 1 weist eine Mehrzahl von Standbeinen 9 auf. Bei dem vorliegenden Ausführungsbeispiel sind drei Standbeine 9 vorgesehen (Dreibein), die sich von dem unteren Rand des Mantelteils 1 radial nach Außen erstrecken. Die Standbeine 9 weisen jeweils einen Standfuß 10 auf. Die Standfüße 10 können an den Standbeinen 9 befestigte, beispielsweise mit den Standbeinen verschraubte Gummifüße sein, mit denen die Vorrichtung sicher auf dem Boden aufsteht und nicht verrutschen oder kippen kann. Da die Vorrichtung mit den Standfüßen 10 auf dem Boden aufsteht, ist die Unterkante des Mantelteils 1 im Abstand zum Boden 11 angeordnet. Dieser Abstand ist in Fig. 3 mit dem Bezugszeichen B bezeichnet.

Die Vorrichtung zum Ableiten von Abwasser wird auf den Boden 11 eines Abwasserbeckens, beispielsweise eines Waschbeckens gestellt. Während des Betriebs der Vorrichtung tritt das Abwasser unter einem relativ hohen Druck, der bei 3 bar liegen kann, aus den Zustromleitungen 6, 7, 8 aus und spritzt auf den Boden 11 des Beckens. Aufgrund der Freifallstrecke, die eine Länge L = A + B hat, wird eine Rückkontamination vermieden. Das Abwasser kann unter dem Mantelteil 1 zu allen Seiten in das Becken abfließen und in den Abfluss des Beckens ablaufen. Spritzwasser wird von dem Deckel- und Mantelteil 1, 2 zurückgehalten und fließt an der Wandung des Mantelteils 1 ab. Eine Rückkontamination des abfließenden Spritzwassers wird dadurch vermieden, dass die Unterkante des Mantelteils 1 im Abstand zu dem Beckenboden 11 angeordnet ist, wodurch eine Freifallstrecke mit der Länge L = B ausgebildet wird. Das Spritzwasser kann an der Unterkante des Mantelteils 1 über dessen gesamten Umfang abfließen oder abtropfen und unter dem Mantelteil in den Abfluss ablaufen.

Fig. 4 zeigt ein zweites Ausführungsbeispiel der Vorrichtung in geschnittener Darstellung, das sich von der ersten Ausführungsform dadurch unterscheidet, dass die Zustromleitungen 6, 7, 8 unterschiedliche Längen l₁, l₂ haben. Die einander entsprechenden Teile sind mit den gleichen Bezugszeichen versehen. Die mittlere Zustromleitung 6 hat eine größere Länge l₁ als die äußeren Zustromleitungen. Folglich ergeben sich Freifallstrecken unterschiedlicher Längen L₁, L₂.

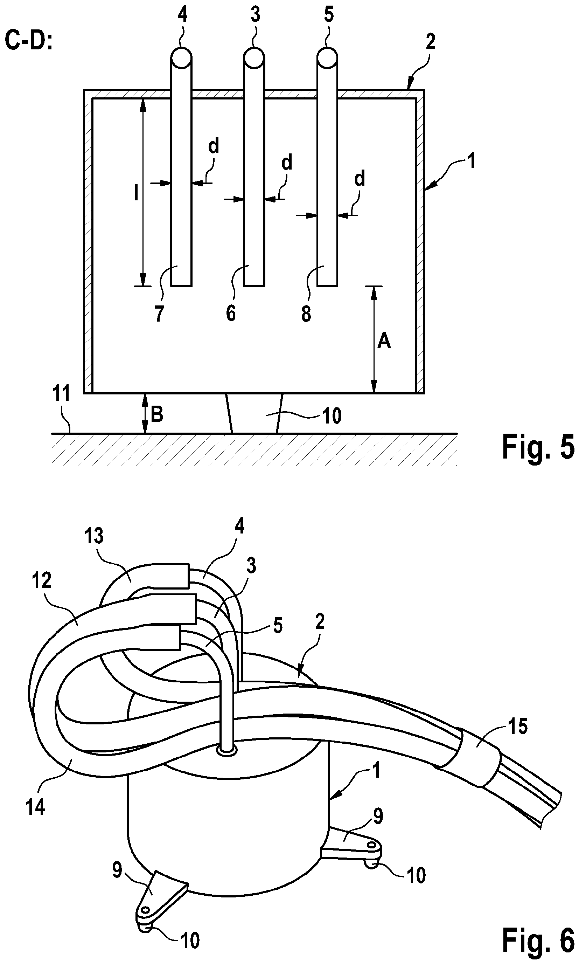

Fig. 5 zeigt ein drittes Ausführungsbeispiel der Vorrichtung in geschnittener Darstellung, das sich von der ersten Ausführungsform dadurch unterscheidet, dass die Zustromleitungen 6, 7, 8 den gleichen Durchmesser d und die gleiche Länge 1 haben. Die einander entsprechenden Teile sind mit den gleichen Bezugszeichen versehen.

Fig. 6 zeigt eine perspektivische Darstellung der Vorrichtung zum Ableiten von Brauchwasser, wobei die Abwasserschläuche 12, 13, 14 eines nicht dargestellten medizintechnischen Geräts an den Anschlussstutzen 3, 4, 5 angeschlossen sind. Der mittlere Abwasserschlauch 12 hat einen größeren Schlauchdurchmesser als die äußeren Abwasserschläuche 13, 14. Die seitlich an die Anschlussstutzen 3, 4, 5 angeschlossenen Abwasserschläuche 12, 13, 14 sind in einem halbkreisförmigen Bogen über den Deckelteil 2 zurückgeführt und mit einem Klettband 15 oder dergleichen zu einem Schlauchbündel zusammengebunden. Die Vorrichtung dient somit auch der Führung und Fixierung der Abwasserschläuche 12, 13, 14. Die Abwasserschläuche 12, 13, 14 können sich aber auch zu der anderen Seite erstrecken, ohne einen halbkreisförmigen Bogen zu bilden.

Mit der erfindungsgemäßen "Abfluss-Glocke" kann für mehrere medizintechnische Geräte gleichzeitig ein sicherer, stabiler Abfluss hergestellt werden. Die Abfluss-Glocke kann in jedem Waschbecken platziert werden. Die Führung und Fixierung der Abwasserschläuche stellt einen kontrollierten Ablauf des Abwassers sicher, wobei die Freifallstrecken eine Rückkontamination in den jeweiligen Abwasserschlauch verhindern. Als Schweißteil kann die Abfluss-Glocke aufgrund der einfachen geometrischen Form ohne hohe Anforderungen an die Fertigungstechnik, insbesondere die Maßhaltigkeit, kostengünstig hergestellt werden. Die Abfluss-Glocke ist ohne Werkzeuge frei zugänglich und lässt sich einfach reinigen bzw. desinfizieren. Da das Abwasser zu allen Seiten nach unten abfließen kann, ist das Abwasser im Becken gut sichtbar, so dass dessen optische Beschaffenheit überwacht werden kann. Die Abfluss-Glocke ist nicht nur in der Medizintechnik, sondern in sämtlichen Bereichen universell einsetzbar, wenn Flüssigkeiten in einen Ablauf abgeleitet werden sollen.

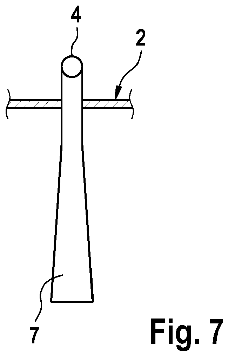

Fig. 7 zeigt eine Zustromleitung 7 der Vorrichtung zum Ableiten von Abwassser, insbesondere Dialysierflüssigkeit. Die Zustromleitung 7 ist als Diffusor ausgebildet und weist einen zum Ende hin sich vergrößernden Querschnitt auf, so dass das Abwasser abgebremst wird und mit einer geringeren Geschwindigkeit in die Freifallstrecke eintritt, beispielsweise mit 1 m/s oder weniger. Dadurch wird unerwünschtes Spritzen zumindest vermindert oder verhindert.

## Patentansprüche

1. Vorrichtung zum Ableiten von Abwasser eines medizintechnischen Gerätes, welche eine Mehrzahl von Leitungsanschlüssen (3, 4, 5) zum Anschluss von Abwasserleitungen des medizintechnischen Gerätes aufweist, **dadurch gekennzeichnet, dass** die Vorrichtung zum Ableiten von Abwasser als ein auf den Boden eines Beckens aufstellbares Teil ausgebildet ist, wobei die Leitungsanschlüsse (3, 4, 5) mit mindestens einer Zustromleitung (6, 7, 8) in Flüssigkeitsverbindung stehen, deren unteres Ende unter Bildung einer Freifallstrecke im Abstand zu der Unterseite des auf den Boden des Beckens aufstellbaren Teils angeordnet ist, wobei die Vorrichtung zum Ableiten von Abwasser ein Mantelteil (1) aufweist, das die mindestens eine sich in das Innere des Mantelteils (1) erstreckende Zustromleitung (6, 7, 8) umschließt.

2. Vorrichtung zum Ableiten von Abwasser nach Anspruch 1, **dadurch gekennzeichnet, dass** das Mantelteil (1) eine Mehrzahl von seitlich abstehenden Standbeinen (9) aufweist.

3. Vorrichtung zum Ableiten von Abwasser nach Anspruch 2, **dadurch gekennzeichnet, dass** die Standbeine (9) Standfüße (10) aufweisen, so dass die Unterseite des Mantelteils (1) im Abstand zum Boden des Beckens angeordnet ist.

4. Vorrichtung zum Ableiten von Abwasser nach Anspruch 2 oder 3, **dadurch gekennzeichnet, dass** der Mantelteil (1) ein hohlzylindrischer Teil ist.

5. Vorrichtung zum Ableiten von Abwasser nach einem der Ansprüche 1 bis 4, **dadurch gekennzeichnet, dass** die Vorrichtung zum Ableiten von Abwasser ein Deckelteil (1) aufweist, an dem die Leitungsanschlüsse (3, 4, 5) vorgesehen sind.

6. Vorrichtung zum Ableiten von Abwasser nach Anspruch 5, **dadurch gekennzeichnet, dass** der Deckelteil (1) ein kreisförmiger Teil ist.

7. Vorrichtung zum Ableiten von Abwasser nach einem der Ansprüche 1 bis 6,
**dadurch gekennzeichnet, dass** die mindestens eine Zustromleitung (6, 7, 8) ein Röhrchen ist, dessen oberes Ende als Anschlussstutzen (3, 4, 5) zum Anschluss einer Schlauchleitung ausgebildet ist.

8. Vorrichtung zum Ableiten von Abwasser nach Anspruch 7, **dadurch gekennzeichnet, dass** der Anschlussstutzen (3, 4, 5) abgewinkelt ist.

9. Vorrichtung zum Ableiten von Abwasser nach einem der Ansprüche 1 bis 8,
**dadurch gekennzeichnet, dass** ein Leitungsanschluss (3, 4, 5) jeweils mit einer Zustromleitung in Fluidverbindung steht.

10. Vorrichtung zum Ableiten von Abwasser nach Anspruch 9, **dadurch**
**gekennzeichnet, dass** die Zustromleitungen (6, 7, 8) die gleiche Länge haben, so dass die unteren Enden der Zustromleitungen (6, 7, 8) den gleichen Abstand zu der Unterseite des auf den Boden des Beckens aufstellbaren Teils haben.

11. Vorrichtung zum Ableiten von Abwasser nach Anspruch 9, **dadurch**
**gekennzeichnet, dass** die Zustromleitungen (6, 7, 8) unterschiedliche Längen haben, so dass die die unteren Enden der Zustromleitungen (6, 7, 8) unterschiedliche Abstände zu der Unterseite des auf den Boden des Beckens aufstellbaren Teils haben.

12. Vorrichtung zum Ableiten von Abwasser nach einem der Ansprüche 9 bis 11, **dadurch gekennzeichnet, dass** die Zustromleitungen (6, 7, 8) den gleichen Durchmesser haben.

13. Vorrichtung zum Ableiten von Abwasser nach einem der Ansprüche 9 bis 11, **dadurch gekennzeichnet, dass** die Zustromleitungen (6, 7, 8) unterschiedliche Durchmesser haben.

14. Vorrichtung zum Ableiten von Abwasser nach einem der Ansprüche 1 bis 13, **dadurch gekennzeichnet, dass** der Mantelteil (1) und/oder der Deckelteil (2) aus Edelstahl bestehen.

15. Vorrichtung zum Ableiten von Abwasser nach einem der Ansprüche 1 bis 14,
**dadurch gekennzeichnet, dass** die Zustromleitung (6, 7, 8) oder mindestens eine der Zustromleitungen (6, 7, 8) einen Diffusor aufweist oder als Diffusor ausgebildet ist, so dass die Flüssigkeitsströmung verlangsamt wird.

16. Vorrichtung zum Ableiten von Abwassser nach Anspruch 15, **dadurch**
**gekennzeichnet, dass** die Zustromleitung (6, 7, 8) oder eine der Zustromleitungen (6, 7, 8) einen sich zum unteren Ende aufweitenden Querschnitt aufweist.

## Claims

1. Device for discharging wastewater of a medico-technical device, comprising a plurality of line connections (3, 4, 5) for connecting wastewater lines of the medico-technical device, **characterised in that** the device for discharging wastewater is designed as a part which can stand on the bottom of a basin, the line connections (3, 4, 5) being in fluid connection with at least one inflow line (6, 7, 8), the lower end of which is arranged so as to be spaced apart from the underside of the part which can stand on the bottom of the basin, such that a free-fall distance is formed, the device for discharging wastewater comprising a casing (1) which surrounds the at least one inflow line (6, 7, 8) extending inside the casing (1).

2. Device for discharging wastewater according to claim 1, **characterised in that** the casing (1) comprises a plurality of laterally protruding supporting legs (9).

3. Device for discharging wastewater according to claim 2, **characterised in that** the supporting legs (9) comprise supporting feet (10) such that the underside of the casing (1) is arranged so as to be spaced apart from the bottom of the basin.

4. Device for discharging wastewater according to either claim 2 or 3, **characterised in that** the casing (1) is a hollow cylindrical part.

5. Device for discharging wastewater according to any of claims 1 to 4, **characterised in that** the device for discharging wastewater comprises a lid (1) on which the line connections (3, 4, 5) are provided.

6. Device for discharging wastewater according to claim 5, **characterised in that** the lid (1) is a circular part.

7. Device for discharging wastewater according to any of claims 1 to 6, **characterised in that** the at least one inflow line (6, 7, 8) is a cannula, the upper end of which is designed as a connecting piece (3, 4, 5) for connecting a hose line.

8. Device for discharging wastewater according to claim 7, **characterised in that** the connecting piece (3, 4, 5) is angled.

9. Device for discharging wastewater according to any of claims 1 to 8, **characterised in that** each line connection (3, 4, 5) is in fluid connection with one inflow line, respectively.

10. Device for discharging wastewater according to claim 9, **characterised in that** the inflow lines (6, 7, 8) are of the same length, such that the lower ends of the inflow lines (6, 7, 8) are at the same spacing from the underside of the part which can stand on the bottom of the basin.

11. Device for discharging wastewater according to claim 9, **characterised in that** the inflow lines (6, 7, 8) are of different lengths, such that the lower ends of the inflow lines (6, 7, 8) are at different spacings from the underside of the part which can stand on the bottom of the basin.

12. Device for discharging wastewater according to any of claims 9 to 11, **characterised in that** the inflow lines (6, 7, 8) are of the same diameter.

13. Device for discharging wastewater according to any of claims 9 to 11, **characterised in that** the inflow lines (6, 7, 8) are of different diameters.

14. Device for discharging wastewater according to any of claims 1 to 13, **characterised in that** the casing (1) and/or the lid (2) consist of stainless steel.

15. Device for discharging wastewater according to any of claims 1 to 14, **characterised in that** the inflow line (6, 7, 8) or at least one of the inflow lines (6, 7, 8) comprises a diffuser or is designed as a diffuser, so that the fluid flow is slowed down.

16. Device for discharging wastewater according to claim 15, **characterised in that** the inflow line (6, 7, 8) or at least one of the inflow lines (6, 7, 8) comprises a cross-section that expands to the lower end.

## Revendications

1. Dispositif d'évacuation des eaux résiduaires d'un appareil médical, comprenant une pluralité de raccords de conduite (3, 4, 5) destinés à raccorder des conduites d'eaux résiduaires de l'appareil médical,
**caractérisé en ce que** le dispositif d'évacuation des eaux résiduaires est réalisé sous la forme d'une pièce susceptible d'être posée sur le fond d'un bassin, les raccords de conduite (3, 4, 5) étant en communication fluidique avec au moins une conduite d'amenée (6, 7, 8) dont l'extrémité inférieure est disposée à distance de la face inférieure de la pièce susceptible d'être posée sur le fond du bassin, en formant un trajet de chute libre, le dispositif d'évacuation des eaux résiduaires comprenant une partie enveloppe (1) qui entoure ladite au moins une conduite d'amenée (6, 7, 8) s'étendant vers l'intérieur de la partie enveloppe (1).

2. Dispositif d'évacuation des eaux résiduaires selon la revendication 1, **caractérisé en ce que** la partie enveloppe (1) présente une pluralité de jambes de support (9) faisant saillie latéralement.

3. Dispositif d'évacuation des eaux résiduaires selon la revendication 2, **caractérisé en ce que** les jambes de support (9) présentent des pieds de support (10), de sorte que la face inférieure de la partie enveloppe (1) est disposée à distance du fond du bassin.

4. Dispositif d'évacuation des eaux résiduaires selon la revendication 2 ou 3, **caractérisé en ce que** la partie enveloppe (1) est une partie cylindrique creuse.

5. Dispositif d'évacuation des eaux résiduaires selon l'une des revendications 1 à 4,
**caractérisé en ce que** le dispositif d'évacuation des eaux résiduaires comprend une partie couvercle (1) sur laquelle sont prévus les raccords de conduite (3, 4, 5).

6. Dispositif d'évacuation des eaux résiduaires selon la revendication 5, **caractérisé en ce que** la partie couvercle (1) est une partie circulaire.

7. Dispositif d'évacuation des eaux résiduaires selon l'une des revendications 1 à 6,
**caractérisé en ce que** ladite au moins une conduite d'amenée (6, 7, 8) est un tube dont l'extrémité supérieure est conçue comme une tubulure de raccordement (3, 4, 5) destinée à raccorder une conduite en tuyau flexible.

8. Dispositif d'évacuation des eaux résiduaires selon la revendication 7, **caractérisé en ce que** la tubulure de raccordement (3, 4, 5) est coudée.

9. Dispositif d'évacuation des eaux résiduaires selon l'une des revendications 1 à 8,
**caractérisé en ce qu'**un raccord de conduite (3, 4, 5) est en communication fluidique avec une conduite d'amenée respective.

10. Dispositif d'évacuation des eaux résiduaires selon la revendication 9, **caractérisé en ce que** les conduites d'amenée (6, 7, 8) ont la même longueur, de sorte que les extrémités inférieures des conduites d'amenée (6, 7, 8) ont la même distance par rapport à la face inférieure de la pièce susceptible d'être posée sur le fond du bassin.

11. Dispositif d'évacuation des eaux résiduaires selon la revendication 9, **caractérisé en ce que** les conduites d'amenée (6, 7, 8) ont des longueurs différentes, de sorte que les extrémités inférieures des conduites d'amenée (6, 7, 8) ont des distances différentes par rapport à la face inférieure de la pièce susceptible d'être posée sur le fond du bassin.

12. Dispositif d'évacuation des eaux résiduaires selon l'une des revendications 9 à 11,
**caractérisé en ce que** les conduites d'amenée (6, 7, 8) ont le même diamètre.

13. Dispositif d'évacuation des eaux résiduaires selon l'une des revendications 9 à 11,
**caractérisé en ce que** les conduites d'amenée (6, 7, 8) ont des diamètres différents.

14. Dispositif d'évacuation des eaux résiduaires selon l'une des revendications 1 à 13,
**caractérisé en ce que** la partie enveloppe (1) et/ou la partie couvercle (2) sont en acier inoxydable.

15. Dispositif d'évacuation des eaux résiduaires selon l'une des revendications 1 à 14,
**caractérisé en ce que** la conduite d'amenée (6, 7, 8) ou au moins l'une des conduites d'amenée (6, 7, 8) comprend un diffuseur ou est conçue comme un diffuseur, de sorte que l'écoulement du liquide est ralenti.

16. Dispositif d'évacuation des eaux résiduaires selon la revendication 15, **caractérisé en ce que** la conduite d'amenée (6, 7, 8) ou l'une des conduites d'amenée (6, 7, 8) présente une section transversale s'élargissant vers l'extrémité inférieure.
